# EUROPÄISCHE PATENTANMELDUNG

(11) **EP 0 853 000 A2**
(43) Veröffentlichungstag der Anmeldung: **15.07.1998**
(21) Anmeldenummer: 98100454.2
(22) Anmeldetag: 13.01.1998
(51) Int. Cl.: B32B 27/04, B32B 27/32

(54) **Fasermatrixverstärktes flächiges Gebilde**

(30) Priorität: 13.01.1997 DE 19700855
(71) Anmelder: BP Chemicals PlasTec GmbH, 89165 Dietenheim (DE)
(72) Erfinder: Dirmeier, Oliver, 83512 Wasserburg (DE); Kauschke, Michael, 83253 Rimsting (DE)
(74) Vertreter: Dost, Wolfgang, Dr.rer.nat., Dipl.-Chem.

(57) **Zusammenfassung**

Die Erfindung betrifft ein fasermatrixverstärktes flächiges Gebilde, umfassend eine Fasermatrix und einen Polymerfilm; wobei auf der einen Oberfläche des Gebildes (Filmseite) die Fasermatrix somit Polymer des Polymerfilms überzogen ist, daß das Relief der Oberfläche der Fasermatrix ausgebildet ist; wobei die mittlere Eindringtiefe des Polymers ausgehend von der Filmseite 1/4 bis 4/4 der Dicke des Gebildes beträgt; wobei mindestens 1 % der die mittlere Eindringtiefe bildendenden Einzeleindringtiefen von der Filmseite bis zur gegenüberliegenden Oberfläche (Fasermatrixseite) des Gebildes reichen sowie ein Verfahren zu dessen Herstellung als auch dessen Verwendung für medizinische Pflaster, für Verpackungsmaterial, als Windel- oder Medikalfolie oder Folie im Damenhygienebereich sowie für Betteinlagen, Operationsabdeckungen und Bekleidung.

## Beschreibung

Die vorliegende Erfindung betrifft ein fasermatrixverstärktes flächiges Gebilde, ein Verfahren zu seiner Herstellung sowie seine Verwendung, insbesondere für den Hygienebereich und für medizinische Pflaster, für Verpackungsmaterial, als Windel- oder Medikalfolie oder Folie im Damenhygienebereich sowie für Betteinlagen, Operationsabdeckungen und Bekleidung.

Als Laminate für den Hygienebereich, beispielsweise Kinder- und Erwachsenenwindeln sowie Damenprodukte, und für medizinische Zwecke, beispielsweise Pflaster und Wundverbände sowie bei Betteinlagen oder Operationsabdeckungen sind bereits zahlreiche Materialien auf Folien-, Gewebe-, Gewirke- oder Vlies-Basis bekannt und auch in der Praxis eingesetzt. Da die Produkte in der Regel eine ganze Reihe von Anforderungen erfüllen müssen, indem sie zwar weich und anschmiegsam sein sollen, je nach Verwendungszweck bevorzugt mit "textilem Griff", aber auch - je nach Einsatzgebiet genügend stabil und widerstandsfähige sein müssen, um beispielsweise den Wundbereich genügend zu schützen, oder im Bereich der Windel entsprechende Exkremente sicher aufzunehmen und einzuschließen. wurden im Stand der Technik verschiedene mehrschichtige Produkte vorgeschlagen. Derartige Laminate sind beispielsweise in den EP-A 45 592, EP- A 81 990, EP-A 257 133, WO 85/05322, WO 85/05322, WO 85/05373, WO 89/01345, US-A 4 727 868 und der US-A 4 751 133 beschrieben. Sie werden durch Tauchen oder direktes Verbinden der einzelnen Trägermaterialien oder mittels einer Klebeschicht hergestellt und weisen dabei den Nachteil auf, daß entweder der angenehme "textile Griff" verloren geht, wenn beispielsweise beim Tauchen das Fasergewebe ganz mit Polymermasse umgeben wird, wie in der US-A 4 727 868 beschrieben, oder daß durch Zusammenkaschieren mit oder ohne Klebeschicht der Schichtaufbau einen großen Teil seiner Flexibilät und Geschmeidigkeit verliert.

DE 34 39 526 offenbart ein Verfahren, bei dem ein Verbundvlies aus einem Spinnvlies und einer Kunststoffbahn mit Hilfe eines Walzenpaares hergestellt wird. Dabei wird in den Spalt, der aus dem Vlies und einer Walze des Walzenpaares gebildet wird, die aus einer Breitschlitzextruderdüse austretende Kunststoffbahn eingeführt und mit dem durch das Walzenpaar transportierten Vlies unter Druckanwendung bei einer Temperatur im oder oberhalb des Schmelzbereiches vereinigt.

Ein Nachteil dieses Verfahrens besteht darin, daß die Walzen die Kunststoffbahn sehr tief in das Vlies einpressen. Dieses führt bei dünnen Vliesen mit einer Flächenmasse von 10 bis 20 g/m² dazu, daß praktisch die gesamte Schichtdicke des Vlieses mit Kunststoff durchsetzt ist. Dieses beeinträchtigt die Geschmeidigkeit und Elastizität dieser Verbundvliese stark, was den Einsatzbereich und die Verwendungsmöglichkeiten derartiger Verbundvliese in beträchtlichem Umfang schmälert.

Weiterhin ist es bei diesem Verfahren nicht möglich, das Relief der Oberfläche der Fasermatrix in dem Verbundvlies auszubilden. Eine dem Relief der Oberfläche des Vlieses entsprechende Struktur kann in diesem Verfahren nur durch die Verwendung von mindestens einer mit entsprechenden zur Simulation des Reliefs der Oberfläche des Vlieses geeigneten Prägungen versehen Walze erreicht werden, wobei es sich dabei nicht um eine Oberfläche handelt, die mit der Vlieses identisch ist.

Um jedoch die Oberfläche unterschiedlicher Vliese auf dem Verbundvlies ausbilden zu können, müssen je nach Vlies unterschiedlich geprägte Walzen verwendet werden. Dieses ist aufgrund der mit der Herstellung und Bevorratung unterschiedlich geprägter Walzen und deren Ein- und Ausbau in Abhängigkeit des verwendeten Vlieses besonders aufwendig, mit erheblichen Kosten verbunden und daher besonders nachteilhaft.

Aus DE 40 16 348 C2 ist ein Verfahren zur Herstellung eines Verbundvlieses aus einem Vlies und einer Bahn aus einem polymeren Stoff bekannt, bei dem das Vlies und der polymere Stoff miteinander verbunden sind, in dem das Vlies direkt einer Unterdruckbeschichtungsvorrichtung zugeführt wird, in der die Bahn aus dem polymeren Stoff in noch schmelzflüssigem Zustand auf das Vlies mit geringer Eindringtiefe des polymeren Stoffes auf das Vlies aufgebracht wird, wodurch das Vlies gleichzeitig verfestigt wird. Das durch dieses Verfahren hergestellte Verbundvlies besitzt jedoch in Abhängigkeit zum Flächengewicht des Verbundvlieses ungünstige Materialeigenschaften, wobei vor allen Dingen die Prägehöhe, Zugfestigkeit, Reißfestigkeit, Kraft bei Dehnung zu gering und die Reißdehnung zu hoch sind.

Darüber hinaus gelingt es bei diesem Verfahren nicht, das Relief der Oberfläche des Vlieses so auszubilden, daß diese einen "textilen Griff" aufweist und sich ähnlich dem verwendeten Vlies und nicht wie eine glatte Kunststoff-Folie beim Angreifen anfühlt.

Ferner wird bei der Unterdruck-Beschichtungsvorrichtung eine Unterdruckwalze zur Herstellung des Verbundvlieses eingesetzt. Der Betrieb dieser Unterdruckwalze ist mit einem erheblichen technischen Aufwand verbunden. Insbesondere ist es aufwendig, eine möglichst gleichmäßige Verteilung des Unterdrucks an der Oberfläche dieser Walze zu gewährleisten. Aufgrund der Schwierigkeit, die gleichmäßige Unterdruckverteilung zu gewährleisten, treten bei dem durch dieses Verfahren hergestellten Verbundvliesen oftmals Fehlstellen auf. Dieses führt zu einer Inhomogenität des hergestellten Verbundvlieses und damit zu einem vergleichsweise hohen Anteil an Ausschuß.

Die im Stand der Technik bekannten und durch die zuvor genannten Verfahren des Standes der Technik erhältlichen Produkte haben weiterhin gemein, daß sie aus zwei oder mehr im wesentlichen voneinander getrennt vorliegenden Schichten bestehen. Weiterhin ist nachteilhaft, daß diese Produkte entweder nur eine durch Prägung simuliertes Relief der Fasermatrixoberfläche aufweisen oder die Oberfläche der Fasermatrix in dem Produkt so ausgebildet ist, daß die Haken von Klettverschluß-Systemen in die Fasermatrix eingreifen und in besonders nachteilhaften Fällen Bestandteile der Fasermatrix, beispielsweise Fasern der als Fasermatrix verwendeten Vliese, aus dem Produkt lösen oder sogar entfernen. Weiterhin lassen sich bei den Produkten des Standes der Technik gleichfalls Bestandteile der Fasermatrix durch Anhaften an eine klebende Oberfläche aus dem Produkt lösen oder entfernen. Das Herauslösen und Entfernen führt zu einer Schwächung des Verbundes.

Aufgabe der vorliegenden Erfindung war es demgemäß, ein fasermatrixverstärktes flächiges Gebilde zur Verfügung zu stellen, daß sich insbesondere für den Hygienebereich und für medizinische Zwecke eignet und die vorgenannten Nachteile überwindet.

Ferner lag der Erfindung insbesonders die Aufgabe zugrunde, ein fasermatrixverstärktes flächiges Gebilde zur Verfügung zu stellen, das bei geringem Flächengewicht günstige Materialeigenschaften, insbesondere wie hohe Prägehöhe, Zugfestigkeit, Reißfestigkeit, Kraft bei Dehnung und geringe Reißdehnung aufweist und auf mindestens einer Oberfläche des Gebildes das Relief der Oberfläche der Fasermatrix ausgebildet ist und auf dieser Oberfläche des Gebildes weder die Haken von Klettverschluß-Systemen eingreifen noch klebende Oberflächen oder die Haken Fasermatrixbestandteile aus dem Produkt lösen oder entfernen.

Diese Aufgabe wird erfindungsgemäß durch ein fasermatrixverstärktes flächiges Gebilde, umfassend eine Fasermatrix und einen Polymerfilm; wobei auf der einen Oberfläche des Gebildes (Filmseite) die Fasermatrix so mit Polymer des Polymerfilms überzogen ist, daß das Relief der Oberfläche der Fasermatrix ausgebildet ist; wobei die mittlere Eindringtiefe des Polymers ausgehend von der Filmseite 1/4 bis 4/4, bevorzugt 1/4 bis 3/4 und besonders bevorzugt 3/8 bis 6/8 der Dicke des Gebildes beträgt; wobei mindestens 1, bevorzugt mindestens 10 und besonders bevorzugt mindestens 30 % der die mittlere Eindringtiefe bildenden Einzeleindringtiefen von der Filmseite bis zur gegenüberliegenden Oberfläche (Fasermatrixseite) des Gebildes reichen. Bei einem Gebilde mit besonders vorteilhaften Eigenschaften ist die Fasermatrix im wesentlichen vollständig vom Polymer des Polymerfilms durchdrungen.

Erfindungsgemäß wird unter mittlerer Eindringtiefe die Summe der Einzeleindringtiefen entlang einer gedachten Linie im betrachteten Querschnitt geteilt durch die Anzahl der Einzeleindringtiefen verstanden, wobei die Eindringtiefe sich aus der Strecke zwischen der Oberfläche des matrixverstärkten flächigen Gebildes - im folgenden als Gebilde bezeichnet - und der Polymerfront des Polymer des Polymerfilms ergibt.

Weiterhin ist es für das erfindungsgemäße Gebilde bevorzugt, daß 0,5 bis 20, bevorzugt 1 bis 15 und besonders bevorzugt 2 bis 10 Gew.-% des Polymers des Polymerfilms oberhalb der Fasermatrixoberfläche die Polymerschicht der Filmseite bilden und 99,5 bis 80, bevorzugt 99 bis 85 und besonders bevorzugt 98 bis 90 Gew.-% des Polymers des Polymerfilms sich in der Fasermatrix befinden. Unter Fasermatrixoberfläche wird hier die Fläche verstanden, die sich bei der Draufsicht auf die Fasermatrix ergibt.

Bezüglich der Auswahl der erfindungsgemäßen Fasermatrix bestehen keine besonderen Beschränkungen. Erfindungsgemäß besteht die Fasermatrix insbesondere aus Vliesstoffen, Mull, Gewebe oder Gewirke sowie Netze oder Gittertüll, vorzugsweise aus Vliesstoffen, die vorzugsweise ein Stapelfaser-oder ein Spinnvlies sind.

Das Flächengewicht der erfindungsgemäß eingesetzten Fasermatrix, insbesondere der eingesetzten Vliesstoffe, liegt im Bereich von 5 bis 60, bevorzugt 5 bis 30 und besonders bevorzugt von 10 bis 20 g/m². Typische Faser/Filament-Durchmesser liegen im Bereich von 10-30 µm.

Weiterhin kann die erfindungsgemäße Fasermatrix und insbesondere die erfindungsgemäßen Vliesstoffe aus Naturfasern, wie Baumwolle oder Zellulose oder Kunstfasern, wie Azetatseite, Polyeester, Polyamide, Polyacrylate, Polyurethane, Synthesekautschuke, Polyolefine oder Gemische davon, bestehen. Erfindungsgemäß bevorzugt ist es, wenn die Fasermatrix und insbesondere die Vliesstoffe aus Polyolefinen, vorzugsweise Polyethylen und Polypropylen, besonders bevorzugt aus deren Fasergemischen oder Polymerblends oder Copolymeren besteht.

Erfindungsgemäß kann für die Fasermatrix, insbesondere für den Vliesstoff jedes dem Fachmann bekannte Polymer aus Propylen eingesetzt werden. Insbesondere fallen erfindungsgemäß unter den Begriff Polypropylene, die im wesentlichen kristallin, isotaktisch oder syndiotaktisch sind und vorzugsweise Copolymere des Propylens mit Ethylen, Buten oder anderen α-Olefinen mit einem Comonomergehalt von maximal 10 Mol-%.

Erfindungsgemäß für die Fasermatrix, insbesondere für den Vliesstoff sind alle dem Fachmann bekannten Polyethylene einsetzbar. Darunter fallen vorzugsweise Ethylenhomopolymere, die gegebenenfalls mit Propen, Buten, Penten, Hexen, Hepten oder Octen copolymerisiert sind, wie beispielsweise LDPE oder vorzugsweise LLDPE.

Erfindungsgemäß besonders bevorzugt als Fasermatrix eingesetzt wird ein Polypropylen-Spinnvliesstoff mit einem Flächengewicht von 10 bis 20 g/m².

Als Polymerfilm für die Herstellung des erfindungsgemäßen Gebildes sind sind alle dem Fachmann bekannten zu schmelzbaren Polymerfilmen verarbeitbaren Polymermaterialien geeignet. Erfindungsgemäß bevorzugte Polymerfilme bestehen aus Thermoplasten, thermoplastischen Elastomeren, Synthesekautschuk enthaltenen Copolymeren, beispielsweise ein Styrol-Butadien-Styroloder ein Acryl-Butadien-Styrolcopolymerisat, insbesondere Ethylen-Vinylacetat-Copolymer, einem Polybutylenterephthalat, Polyethylenterephthalat oder seinen Copolymeren, Polyurethanen oder aus deren Gemischen, vorzugsweise aus Polyolefinen oder Polyolefin-Copoylmeren, insbesondere aus Polyethylen, Polyethylen-Copolymer, Polypropylen oder Polypropylen-Copolymeren, wobei Mischungen aus Polyethylen und Polypropylen insbesondere bevorzugt sind. Als Erfindungsgemäß besonders geeignete Polyethylene und Polypropylene kommen die im Zusammenhang mit der Fasermatrix beschriebenen gleichfalls in Betracht. Darüber hinaus sind für den Polymerfilm Polyethylen-Propylen-Blends bevorzugt. Darüberhinaus sind Blends aus Polyethylen-Propylen-Copolymeren mit Polyethylen und/oder Polypropylen bevorzugt. Darüberhinaus sind Blends aus Polyethylen-Propylen-Copolymeren mit Polyethylen und/oder Polypropylen jeweils mit Ethylen-Vinylacetat-Copolymeren besonders bevorzugt.

Erfindungsgemäß besonders bevorzugt ist ein Gebilde, bei dem ein Polymerfilm aus einem Blend aus Polyethylen und Polypropylen eingesetzt wird. In diesem Blend können 10 bis 100 Gew.-% Polypropylen mit 90 bis 0 Gew.-% Polyethylen, bevorzugt 20 bis 90 Gew.-% Polypropylen mit 80 bis 10 Gew.-% Polyethylen und besonders bevorzugt 40 bis 60 Gew.-% Polypropylen mit 60 bis 40 Gew.-% Polyethylen eingesetzt werden, vorzugsweise mit 1 bis 15 % Gew.-% eines Ethylen-Vinylacetat-Copolymers, jeweils bezogen auf das Blend.

Weitere erfindungsgemäß bevorzugte Polymerfilme bestehen aus Polykondensaten, beispielsweise Polyester wie PET, PTD oder Polyamiden, beispielsweise Nylon 66 oder Nylon 6.

Daneben ist es erfindungsgemäß bevorzugt, sowohl für die Fasermatrix als auch für den Polymerfilm jeweils ein mikroporöses wasserdampfpermeables, jedoch wasserdichtes Polymer einzusetzen. Solche mikroporösen Polymere können Homopolymere, Polymerblends, Copolymere oder Polymercompounds (Polymer und geeignete Additive und Füllstoff) sein, wie beispielweise als PE-Compound gemäß Europäischer Patentschrift 038 0353 B1 offenbart.

Für die Fasermatrix, insbesondere für den Vliesstoff, und den Polymerfilm, können gleichfalls biologisch abbaubare Stoffe, beispielsweise auf Cellulosebasis, Viskosebasis, aus Gelatine in Faser- bzw. Filmform, Poly-ε-Caprolacton, Polylactide, lineare Polyester aus 3-Hydroxybuttersäure und 3-Hydroxyvalerinsäure oder dergleichen verwendet werden.

Üblicherweise werden die erfindungsgemäßen Gebilde gemäß ihres Einsatzzweckes, in der Regel weiß mit Titandioxid, eingefärbt. Weiterhin können die erfindungsgemäßen Polymerfilme die dem Fachmann bekannten Zusatzstoffe und Verarbeitungshilfsmittel enthalten.

Die Dicke des zur Herstellung des erfindungsgemäßen Gebildes verwendeten Polymerfilms unterliegt an sich keiner Beschränkung. Es ist jedoch erfindungsgemäß bevorzugt, einen Polymerfilm mit einer Dicke von mindestens 1/8 bis 7/8, bevorzugt 1/8 bis 3/4 und besonders bevorzugt von 1/4 bis 3/4 der Dicke der Fasermatrix zur Herstellung des erfindungsgemäßen Gebildes einzusetzen. Das Flächengewicht des Polymerfilms liegt im Bereich von 3 bis 50, bevorzugt von 3 bis 30 und besonders bevorzugt von 3 bis 20 g/m². Typische Polymerfilmdicken liegen im Bereich von 3-20 µm.

Weiterhin ist es erfindungsgemäß bevorzugt, daß die Fasermatrix, insbesondere der Vliesstoff, und der Polymerfilm Anteile gleichen Polymermaterials aufweisen. Durch diese Maßnahme läßt sich die Haftung von Fasermatrix und Polymerfilm verbessern. Sie bestehen mindesten zu einem Viertel, bevorzugt mindestens zur Hälfte und besonders bevorzugt ganz aus Anteilen gleichen Polymermaterials.

Ferner ist es erfindungsgemäß bevorzugt, daß das Gebilde auf der Fasermatrixseite nur teilweise Polymermaterial aufweist. Dieses bedeutet, daß höchstens 95, bevorzugt höchstens 50 und besonders bevorzugt höchstens 1 % der Fläche der Fasermatrixseite Polymermaterial aufweist.

Weiterhin ist ein Gebilde erfindungsgemäß bevorzugt, insbesondere wenn es aus einem Polypropylenvliesstoff und einem Polyethylen-Polypropylen-Blend Polymerfilm gebildet ist, wenn es bei einem Flächengewicht von 10 bis 60 g/m², bevorzugt 10 bis 40 g/m² und besonders bevorzugt von 10 bis 30 g/m² mindestens eine, bevorzugt mindestens zwei und besonders bevorzugt mindestens drei der in Tabelle 1 aufgeführten Eigenschaften aufweist.

Erfindungsgemäß besonders bevorzugt ist ein Gebilde, das aus einem Polypropylenvliesstoff und einem Polyethylen/Polypropylen-Blend Polymerfilm gebildet ist, wenn es bei einem Flächengewicht von 10 bis 35 g/m² die in Tabelle 1 aufgeführten bevorzugten Eigenschaften aufweist.

Erfindungsgemäß besonders bevorzugt ist ein Gebilde, das aus einem Polypropylenvliesstoff und einem Polyethylen/Polypropylen-Blend Polymerfilm gebildet ist, wenn es bei einem Flächengewicht von 10 bis 35 g/m² die in Tabelle 1 als besonders bevorzugt aufgeführten Eigenschaften aufweist.

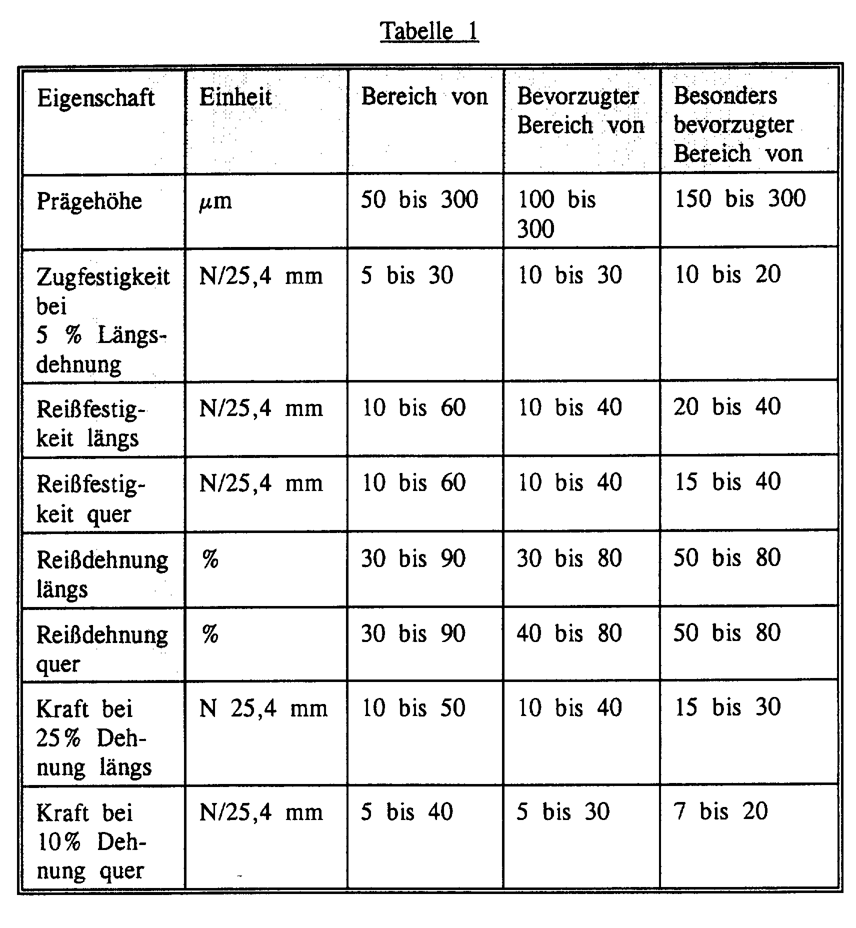

Weiterhin weist das erfindungsgemäße Gebilde vorzugsweise die Eigenschaft auf, daß auf der Filmseite das Relief der Oberfläche der Fasermatrix durch Überziehen mit Polymer des Polymerfilms dann ausgebildet ist, wenn die Ansicht, insbesondere die Grauwerte des Abdrucks der Filmseite des Gebildes der Ansicht, insbesondere den Grauwerten des Abdrucks des Reliefs der Oberfläche der nicht überzogenen Fasermatrix im wesentlichen übereinstimmen und wenn keine Haken von Klettverschluß-Systemen eingreifen können und sich vorzugsweise keine Fasermatrixanteile durch Haken von Klettverschluß-Systemen oder von klebenden Oberflächen aus dem Gebilde lösen oder entfernen lassen, die sich bei gleicher Behandlung aus der nicht mit Polymerfilm in erfindungsgemäßer Weise in Kontakt gebrachten Fasermatrix lösen oder entfernen lassen. Unter Fasermatrixanteilen werden beispielsweise bei Vliesstoffen deren Fasern verstanden.

Die zuvor beschriebene Einwirkung der Haken von Klettverschluß-Systemen und/oder von klebenden Oberflächen auf die Filmseite des erfindungsgemäßen Gebildes kann vorzugsweise dadurch unterbunden werden, wenn die auf der Filmseite befindlichen Fasern der Fasermatrix mindestens mit einem Fünftel, bevorzugt mit einem Drittel und besonders bevorzugt mit der Hälfte ihrer Außenfläche entlang der Faser über das Polymer des Polymerfilms mit dem restlichen Gebilde verbunden sind. Die ausgebildete Polymerschicht, die einerseits durch die Filmseite und anderseits durch die Fasermatrixoberfläche begrenzt wird, besitzt eine Dicke, die vorzugsweise den Einzelfaserdurchmesser der Fasern nicht übersteigt.

Im erfindungsgemäßen Verfahren zur Herstellung des Gebildes werden die Fasermatrix und der Polymerfilm zwischen den Walzen mindestens eines Walzenpaares mit Walzen unterschiedlicher Härte in Kontakt gebracht, wobei die Fasermatrix über die harter Walze läuft und der Polymerfilm vorzugsweise zwischen Vlies und weicher Walze einläuft.

Das Walzenpaar wird aus einer harten Walze, bei der zumindest die Oberfläche aus Stahl besteht, und einer weichen Walze, bei der zumindest die Oberfläche aus Gummi besteht, gebildet. Als für das erfindungsgemäße Verfahren geeignete Gummiwalzen kommen die dem Fachmann bekannten Gummiwalzen mit unterschiedlich dicken und weichen Gummimäntel in Betracht. Vorzugsweise weist die Oberfläche der harten Walze eine Shorehärte von 90 bis 110, bevorzugt von 95 bis 100 und zumindest die Oberfläche der weichen Walze eine Shorehärte von 30 bis 90, bevorzugt 30 bis 70 und besonders bevorzugt 40 bis 60 auf.

Die Auswahl der zur Herstellung des erfindungsgemäßen Gebildes geeignetesten Kombination von harter und weicher Walze im Walzenpaar sowie des Anpressdrucks ergibt sich wie folgt:

Die unbehandelte Fasermatrixoberfläche wird mittels einer Rakel mit schwarzer Druckfabe versehen, mit einem Druck von 0,05 N/m² auf ein weißes Glanzpapier über 1 Sekunde gedrückt und wieder entfernt. Nachdem die Fasermatrix mit dem Polymerfilm in Kontakt gebracht woren ist, wird die Filmseite des Produktes dem gleichen Druckverfahren unterzogen. Nach Trocknen der beiden Abdrücke wird der Grauwert mittels einer Opazitätsmessung als L*,a,b-Wert gemäß DIN 6174 oder als Opazitätswert gemäß DIN 53146 bestimmt. Darüberhinaus können die beiden Abdrücke des Reliefs Fasermatrixoberfläche mit den Augen verglichen werden.

Es ist bei dem erfindungsgemäßen Verfahren weiterhin bevorzugt, daß das Walzenpaar einen Spalt ausbildet, in dem Polymerfilm und Fasermatrix miteinander in Kontakt gebracht werden, so daß der Polymerfilm in die Fasermatrix eindringen kann.

Ferner liegt der Polymerfilm im erfindungsgemäßen Verfahren als Schmelze vor, wenn dieser mit der Fasermatrix in Kontakt gebracht wird. Die Schmelze des Polymerfilms wird erfindungsgemäß vorzugsweise durch einen Extruder mit einer Breischlitzdüse erzeugt. Weiterhin ist es bevorzugt, daß der Polymerfilm, insbesondere wenn dieser als Schmelze vorliegt, unter Ausnutzung der Schwerkraft dem Walzenpaar zugeführt wird. Daher ist es erfindungsgemäß bevorzugt, die Öffnung der Breitschlitzdüse, aus der der Polymerfilm als Schmelze austritt, oberhalb des Walzenpaares anzuordnen.

Die Temperatur der Walzenoberflächen des Walzenpaares und der Fasermatrix liegt unterhalb der Schmelztemperatur des Polymerfilms, vorzugsweise im Bereich von 0 und 60, bevorzugt 10 bis 60 und besonders bevorzugt 15 bis 50 °C.

Das erfindungsgemäße Gebilde wird für Pflaster, vorzugsweise als Trägermaterial für medizinische Pflaster und Fixierpflaster verwendet. Ferner eignet sich das flächige Gebilde zur Verwendung als Windel- oder Medikalfolie oder Folie im Damenhygienebereich bzw. für Betteinlagen oder Operationsabdeckungen. Außerdem eigenen sich die erfindungsgemäßen Gebilde als Verpackungsmaterial. Zudem ist die Verwendung des erfindungsgemäßen Gebildes für Bekleidungen, insbesondere für Arbeits- und Schutzbekleidungen, beispielsweise Kittel, Schürzen, Overalls etc. bevorzugt.

Die Erfindung wird nun anhand der folgenden Figuren und Beispiele, die bevorzugte Ausführungsformen der Erfindung darstellen, näher erläutert.

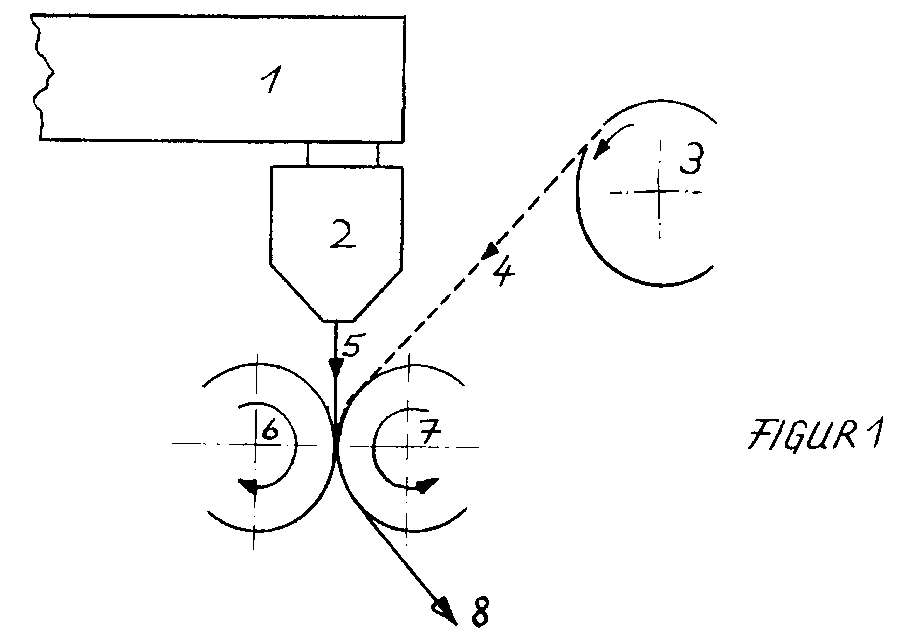

Figur 1 zeigt eine schematische Darstellung einer Vorrichtung, die zur Herstellung des erfindungsgemäßen Gebildes eingesetzt werden kann. Mittels eines Extruders (1) wird aus dem Polymer über eine Breitschlitzdüse (2) der Polymerfilm (5) als Schmelze erzeugt und dem Walzenpaar aus einer weichen Gummiwalze (6) und einer harten Stahlwalze (7) von oben zugeführt. In dem Walzenpaar wird der Polymerfilm (5) mit der Fasermatrix (4) in Kontakt gebracht, wobei die Fasermatrix (4) über einen Abroller (3) zwischen Polymerfilm (5) und Stahlwalze (7) zugeführt wird. Das fertige erfindungsgemäße Gebilde (8) wird unterhalb des Walzenpaares weggeführt.

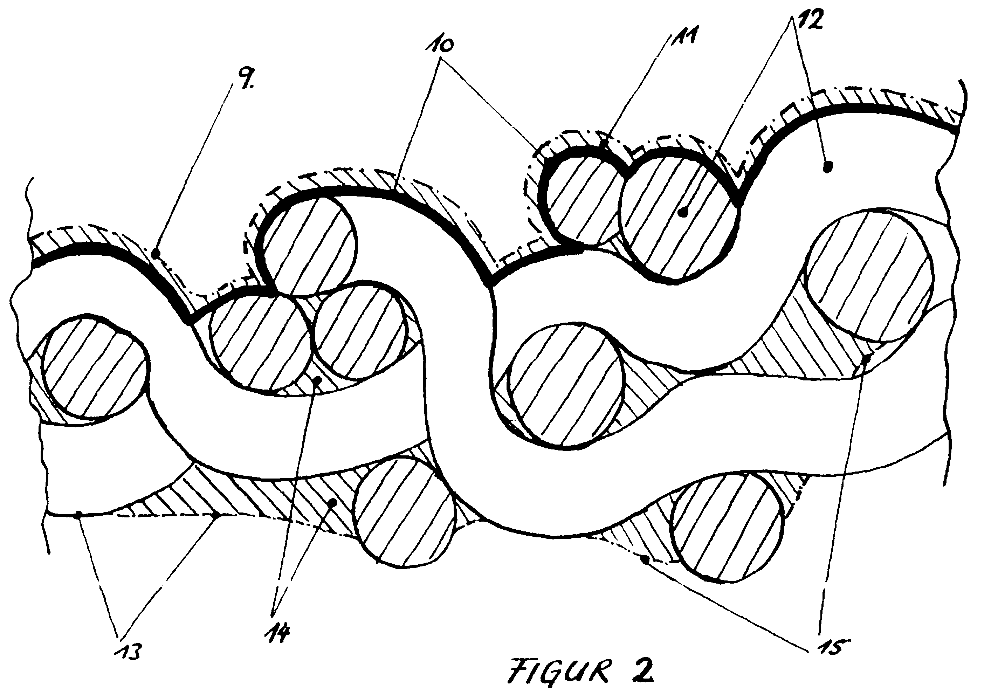

Figur 2 zeigt als Querschnitt die schematische Abbildung eines erfindungsgemäßen Gebildes. Die Filmseite (9) und die Fasermatrixoberfläche (10) bilden die Grenzen des der Polymerschicht (11). Weiterhin weist das erfindungsgemäße Gebilde die Fasern der Fasermatrix (12) und das Polymer (13) auf, wobei das Polymer entsprechend der Eindringtiefe (15) die Fasermatrix teilweise oder ganz bis zur Fasermatrixseite (14) durchdrungen hat.

Figur 3a und 3b zeigt als Querschnittsansicht eine Elektronenrastermikroskopaufnahme des Vergleichsproduktes aus Beispiel 1 in 500-facher Vergrößerung.

Figur 4a und 4b zeigt als Querschnittsansicht eine Elektronenrastermikroskopaufnahme des erfindungsgemäßen Produktes aus Beispiel 2 in 500-facher Vergrößerung.

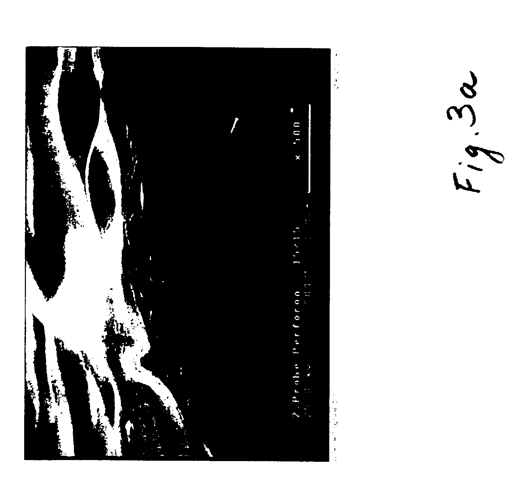

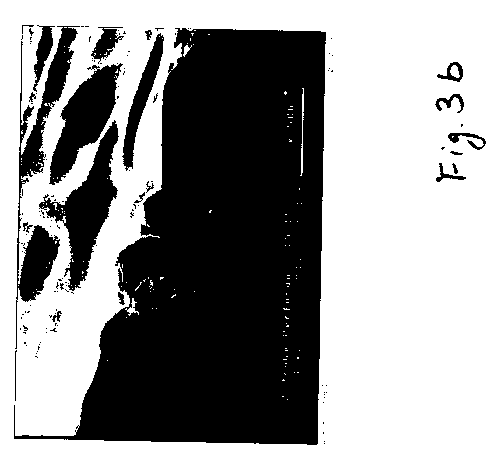

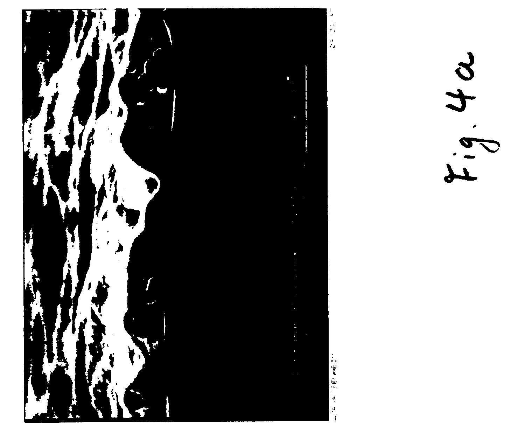

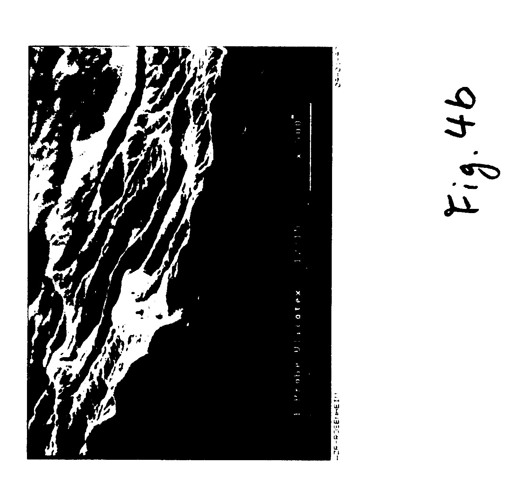

Aus Figur 4a und 4b ergibt sich, daß zum einen auf der Filmseite das Relief der Fasermatrixoberfläche deutlich stärker in dem erfindungsgemäßen Produkt als im Vergleichsprodukt der Figur 3a und 3b ausgebildet ist. Im Gegensatz zum Vergleichsprodukt ist im erfindungsgemäßen das Polymer des Polymerfilms an einigen Stellen teilweise und an anderen ganz in die Fasermatrix eingedrungen. Ferner lösen sich die Fasern der Fasermatrix im Vergleichsprodukt teilweise von der Polymerschicht auf der Filmseite ab, während im erfindungsgemäßen Produkt die Fasern an der Fasermatrixoberfläche mit der Filmschicht überwiegend eine Einheit bilden und fest miteinander verbunden sind.

### BEISPIEL 1

### (Vergle ichsprodukt)

Ein Polypropylen-Spinnvlies mit einem Flächengewicht von 15 g/m² der Firma Carl Freudenberg (CF 9416) wird in einer Unterdruck-Beschichtungsvorrichtung gem. DE 40 16 348 C2, deren mit Bohrungen versehene Stützzylinder eine offene Fläche von etwa 65 % bei einem Bohrungsdurchmesser von 0,4 mm aufweist, und einem Vakuum von 0,1 bis 0,4 x 10⁵ N/m² mit einem durch einen Extruder mit Breitschlitzdüse erzeugten Polymerfilm als Schmelze, der ein Flächengewicht von 12 g/m besitzt und aus einem Polymerblend aus 60 Gew.-% Polypropylen (PP 3520L der Firma BASF) und 30 Gew.-% Low Density Polyethylen (LDPE 3020K der Firma BASF) sowie 4 Gew.-% Ethylen-Vinylacetat-Copolymer (EVA 1040VN4 der Firma ATO) und 6 Gew.-% Weißkonzentrat (APX 600 der Firma Hoechst) besteht, in Kontakt gebracht.

### BEISPIEL 2

### (Erfindungsgemäßes Produkt)

Ein Polypropylen-Spinnvlies mit einem Flächengewicht von 15 g/m² der Firma Carl Freudenberg (CF 9416) wird zwischen den Walzen eines Walzenpaares mit einem durch einen Extruder mit Breitschlitzdüse erzeugten Polymerfilm als Schmelze, der ein Flächengewicht von 12 g/m² besitzt und aus einem Polymerblend aus 60 Gew.-% Polypropylen (PP 3520L der Firma BASF) und 30 Gew.-% Low Density Polyethylen (LDPE 3020K der Firma BASF) sowie 4 Gew.-% Ethylen-Vinylacetat-Copolymer (EVA 1040VN4 der Firma ATO) und 6 Gew.-% Weißkonzentrat (APX 600 der Firma Hoechst) besteht, mit einem Druck von 15 N/mm² in Kontakt gebracht. Das Walzenpaar besteht aus einer auf 25 °C temperierten Stahlwalze und einer 70 Shore A harten ungekühlten Gummiwalze.

Aus dem erfindungsgemäßen Produkt konnten weder durch Abziehen eines zuvor auf der Filmseite aufgebrachten Klebestreifens (Tesafilm der Firma Beiersdorf) noch durch die Haken eines Klettverschluß-Systems (900 - 3000 Haken/in² = 140 - 465 Haken/cm²), das mit der Hand fest auf die Filmseite gedrückt und wieder abgenommen wurde, mit dem bloßen Auge erkennbar Fasermatrixbestandteile gelöst oder entfernt werden. Weiterhin griffen die Haken des Klettverschluß-Systems nicht auf der Filmseite ein, so daß der die Haken tragende Teil des Klettverschlußsystems nicht an der Filmseite haftete.

Die Materialeigenschaften des Vergleichprodukts bei einem Flächengewicht von 30 g/m² und des erfindungsgemäßen Produkts mit einem Flächengewicht von 27,5 g/m², jeweils nach DIN 53352 bestimmt, sind in Tabelle 2 aufgeführt. Die Eigenschaften wurden entsprechend der jeweils angegebenen DIN bestimmt.

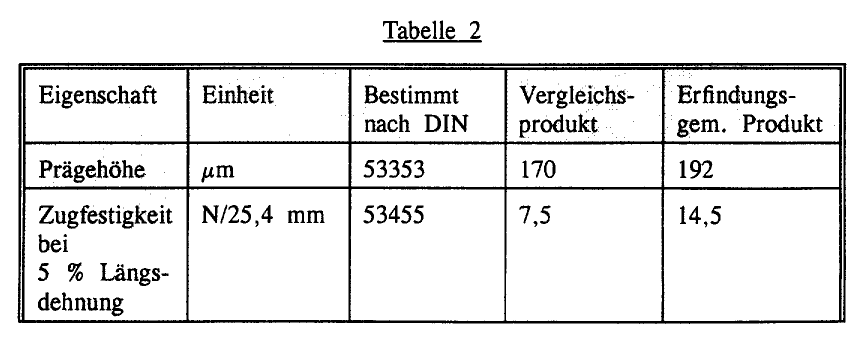

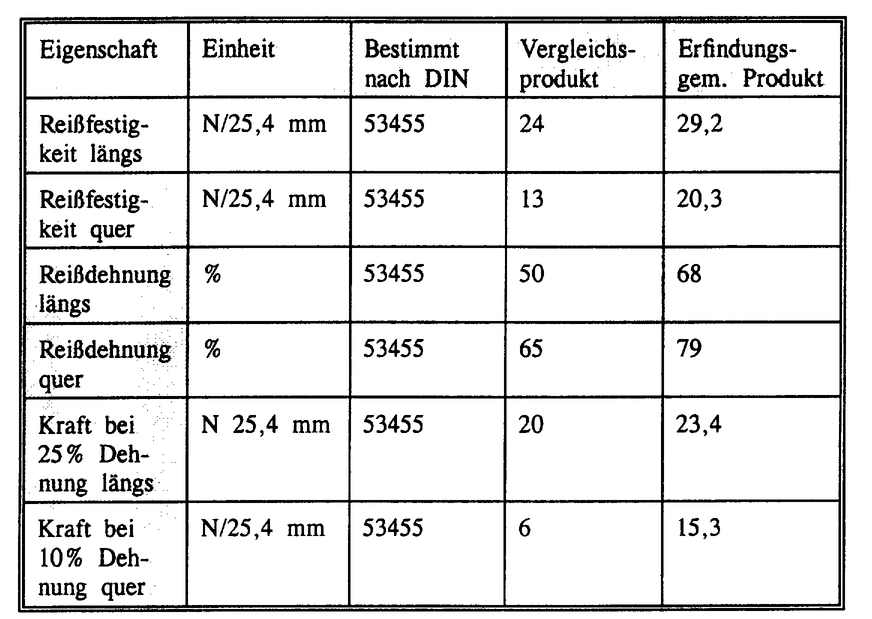

Aus Tabelle 2 wird deutlich, daß das erfindungsgemäße Produkt dem Vergleichsprodukt selbst bei geringerer Flächendichte in allen aufgeführten Materialeigenschaften überlegen ist.

## Patentansprüche

1. Fasermatrixverstärktes flächiges Gebilde, umfassend eine Fasermatrix und einen Polymerfilm; wobei auf der einen Oberfläche des Gebildes (Filmseite) die Fasermatrix so mit Polymer des Polymerfilms überzogen ist, daß das Relief der Oberfläche der Fasermatrix ausgebildet ist; wobei die mittlere Eindringtiefe des Polymers ausgehend von der Filmseite 1/4 bis 4/4 der Dicke des Gebildes beträgt; wobei mindestens 1 % der die mittlere Eindringtiefe bildendenden Einzeleindringtiefen von der Filmseite bis zur gegenüberliegenden Oberfläche (Fasermatrixseite) des Gebildes reichen.

2. Fasermatrixverstärktes flächiges Gebilde nach Anspruch 1, gekennzeichnet, daß 0,5 bis 20 Gew.-% des Polymers des Polymerfilms oberhalb der Fasermatrixoberfläche die Polymerschicht der Filmseite bilden und 99,5 bis 80 Gew.-% des Polymers des Polymerfilms sich in der Fasermatrix befinden.

3. Fasermatrixverstärktes flächiges Gebilde nach einem der vorhergehenden Ansprüche, dadurch gekennzeichnet, daß die Fasermatrix aus Vliesstoff, Mull, Gewebe oder Gewirke, vorzugsweise Vliesstoff besteht.

4. Fasermatrixverstärktes flächiges Gebilde nach einem der vorhergehenden Ansprüche, dadurch gekennzeichnet, daß der Polymerfilm ausgewählt ist aus Thermoplasten, thermoplastischen Elastomeren oder deren Gemischen, vorzugsweise aus Polyolefinen, insbesondere aus Polyethylen oder Polypropylen und vorzugsweise aus deren Polymerblends.

5. Fasermatrixverstärktes flächiges Gebilde nach einem der vorhergehenden Ansprüche, dadurch gekennzeichnet, daß Fasermatrix und Polymerfilm Anteile gleichen Polymermaterials aufweisen.

6. Fasermatrixverstärktes flächiges Gebilde nach einem der vorhergehenden Ansprüche, dadurch gekennzeichnet, daß die Fasermatrix aus einem Vlies aus Polyethylen oder Polypropylen, vorzugsweise Polymerblends hiervon gebildet ist.

7. Fasermatrixverstärktes flächiges Gebilde nach einem der vorhergehenden Ansprüche, dadurch gekennzeichnet, daß die Fasermatrix und der Polymerfilm aus biologisch abbaubaren Materialien bestehen.

8. Fasermatrixverstärktes flächiges Gebilde nach einem der vorhergehenden Ansprüche, dadurch gekennzeichnet, daß die Fasermatrixseite nur teilweise von Polymermaterial durchdrungen ist.

9. Fasermatrixverstärktes flächiges Gebilde nach einem der vorhergehenden Ansprüche, im wesentlichen wie in Figur 2 dargestellt.

10. Fasermatrixverstärktes flächiges Gebilde nach einem der vorhergehenden Ansprüche, dadurch gekennzeichnet, daß es bei einem Flächengewicht von 10 bis 60 g/m² mindestens eine der folgenden Eigenschaften aufweist:

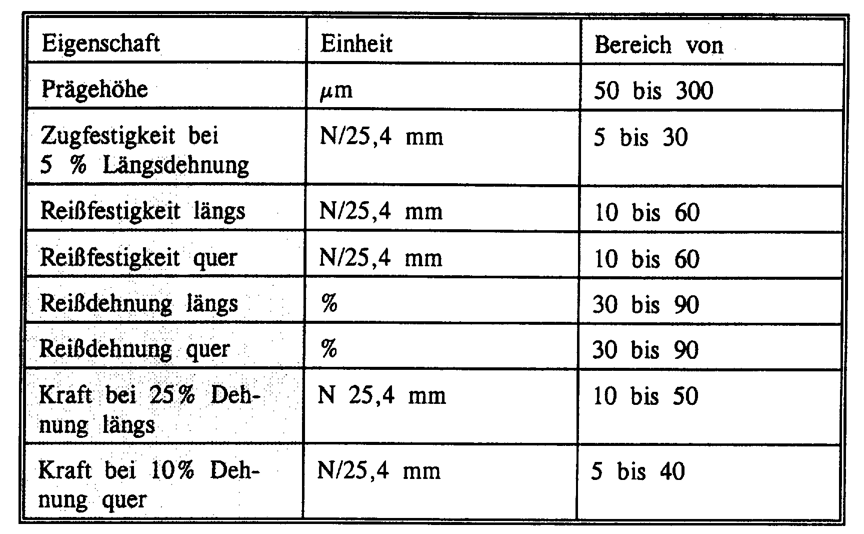

11. Fasermatrixverstärktes flächiges Gebilde nach einem der vorhergehenden Ansprüche, dadurch gekennzeichnet, daß auf der Filmseite das Relief der Oberfläche der Fasermatrix durch Überziehen mit Polymer des Polymerfilms so ausgebildet ist, daß die Ansicht des Abdrucks der Filmseite des Gebildes mit der Ansicht des Abdrucks des Reliefs der Oberfläche der nicht überzogenen Fasermatrix im wesentlichen übereinstimmt, wobei die Abdrücke auf gleiche Weise hergestellt worden sind, und wenn keine Haken von Klettverschluß-Systemen in die Filmseite eingreifen können.

12. Verfahren zur Herstellung eines fasermatrixverstärkten flächigen Gebildes nach einem der vorhergehenden Ansprüche, dadurch gekennzeichnet, daß die Fasermatrix und der Polymerfilm zwischen Walzen mindestens eines Walzenpaares mit Walzen unterschiedlicher Härte in Kontakt gebracht werden, wobei die Fasermatrix über die härtere Walze läuft.

13. Verfahren nach Anspruch 12, dadurch gekennzeichnet, daß das Walzenpaar aus einer ersten Walze, bei der zumindest die Oberfläche aus Stahl besteht, und einer zweiten Walze, bei der zumindest die Oberfläche aus Gummi besteht, gebildet wird.

14. Verfahren nach Anspruch 13, dadurch gekennzeichnet, daß zumindest die Oberfläche der ersten Walze eine Shorehärte von 95 bis 100, und zumindest die Oberfläche der zweiten Walze eine Shorehärte von 20 bis 94 aufweist.

15. Verfahren nach einem der Ansprüche 12 bis 14, dadurch gekennzeichnet, daß das Walzenpaar einen Spalt ausbildet, in dem Polymerfilm und Fasermatrix miteinander in Kontakt gebracht werden.

16. Verfahren nach einem der Ansprüche 12 bis 15, dadurch gekennzeichnet, daß der Polymerfilm als Schmelze mit der Fasermatrix in Kontakt gebracht wird.

17. Verfahren nach einem der Ansprüche 12 bis 16, dadurch gekennzeichnet, daß die Temperatur des Walzenpaares und der Fasermatrix unterhalb der Schmelztemperatur des Polymerfilms liegt.

18. Fasermatrixverstärktes flächiges Gebilde nach einem der Ansprüche 1 bis 11, erhältlich durch ein Verfahren nach einem der Ansprüche 12 bis 17.

19. Verwendung eines fasermatrixverstärkten flächigen Gebildes nach einem der Ansprüche 1 bis 18 als Trägermaterial für medizinische Pflaster.

20. Verwendung eines fasermatrixverstärkten flächigen Gebildes nach einem der Ansprüche 1 bis 18 als Trägermaterial für Pflaster.

21. Verwendung eines fasermatrixverstärkten flächigen Gebildes nach einem der Ansprüche 1 bis 18 als Windel- oder Medikalfolie oder Folie im Damenhygienebereich oder für Betteinlagen oder Operationsabdeckungen.

22. Verwendung eines fasermatrixverstärkten flächigen Gebildes nach einem der Ansprüche 1 bis 18 für Verpackungsmaterial.

23. Verwendung eines fasermatrixverstärkten flächigen Gebildes nach einem der Ansprüche 1 bis 18 für Bekleidungen.
